# EUROPEAN PATENT SPECIFICATION

(11) **EP 1 827 409 B1**
(45) Date of publication and mention of the grant of the patent: **24.08.2011**
(21) Application number: 05851829.1
(22) Date of filing: 17.11.2005
(51) Int. Cl.: A61K 31/135, A61K 47/26, A61P 25/16

(54) **RASAGILINE ORALLY DISINTEGRATING COMPOSITIONS**
IM MUND ZERFALLENDE ZUSAMMENSETZUNGEN VON RASAGILIN
COMPOSITIONS DE RASAGILINE DELITANTES ORALEMENT

(30) Priority: 24.11.2004 US 630918 P; 24.11.2004 US 997785
(43) Date of publication of application: 05.09.2007
(73) Proprietor: TEVA PHARMACEUTICAL INDUSTRIES, LTD., 49131 Petach-Tikva (IL)
(72) Inventor: PATASHNIK, Shulamit, 71908 Reut (IL); LICHT, Daniella, 54041 Givat Shmuel (IL); GILBERT, Adrian, 43465 Ra' Anana (IL)
(74) Representative: Nachshen, Neil Jacob
(86) International application number: PCT/US2005/041882
(87) International publication number: WO 2006/057912

(56) References cited:
- WO-A-96/26714
- WO-A-2006/014973
- WO-A-2006/058250
- US-A- 6 126 968

## Description

### BACKGROUND OF THE INVENTION

U.S. Patent Nos. 5,532,415, 5,387,612, 5,453,446, 5,457,133, 5,599,991, 5,744,500, 5,891,923, 5,668,181, 5,576,353, 5,519,061, 5,786,390, 6,316,504 and 6,630,514, and PCT International Publication Nos. WO 95/11016 and WO 96/37199, disclose R(+)-propargyl-1-aminoindan, also known as rasagiline. Rasagiline has been shown to be a selective inhibitor of the B-form of the enzyme monoamine oxidase, useful in treating Parkinson's disease and various other conditions by inhibition of MAO in the brain.

Pharmaceutical formulations of rasagiline are disclosed in, e.g. WO 95/11016. However, U.S. Patent No. 6,126,968 subsequently disclosed that the formulations of WO 95/11016 were of unacceptable stability, pointing out that Example 20 of WO 95/11016 contained 3.08% degradants after six months of storage. U.S. Patent No. 6,126,968 then proceeds to offer certain alternative formulations of rasagiline intended to provide improved stability relative to the formulations of WO 95/11016. The formulations disclosed in U.S. Patent No. 6,129,968 are for ingestable tablet form compositions.

A major source of concern in using ingestible forms of monoamine oxidase inhibitors such as rasagiline is the risk of hypertensive crises, often called the "cheese effect." (Simpson G.M. and White K., "Tyramine studies and the safety of MAOI drugs", J Clin Psychiatry (1984 Jul) Vol. 45 (7 pt 2), pages 59-91). This effect is caused by inhibition of peripheral MAO. (Id. at page 59). A high concentration of peripheral MAO is found the stomach. (Id. at 59). Therefore, if rasagiline could be administered without being absorbed in the stomach, any cheese effect potential could be avoided.

Furthermore, Parkinsonian patients suffer from swallowing disorders which prevent them from swallowing standard tablets or capsules. (Potulska A., "Swallowing disorders in Parkinson's disease", Parkinsonism Relat. Disord. (2003 Aug) Vol. 9(6), pages 349-53). This difficulty hinders their treatment by reducing patient compliance. Patients will be more likely to comply to dosage regimens if swallowing tablets or capsules is not required.

EP 0 814 789 discloses formulations of MAO-B inhibitors which attempts to address some of the known problems. However, EP 0 814 789 relies on lyophilization of the MAO-B inhibitor formulations which is a costly process and results in high friability of the product, further increasing cost by necessitating costly special blister-pack packaging.

### SUMMARY OF THE INVENTION

This invention provides a solid pharmaceutical composition comprising rasagiline or a pharmaceutically acceptable salt of rasagiline, and particles having a non-filamentous microstructure of at least two sugar alcohols.

This invention also provides a solid pharmaceutical composition comprising rasagiline or a pharmaceutically acceptable salt of rasagiline, a mixture of a disintegrant, a flow agent and particles having a non-filamentous microstructure of at least two sugar alcohols, a supplemental sugar alcohol, a supplemental flow agent, and a supplemental disintegrant.

This invention also provides a solid pharmaceutical composition comprising 0.9% rasagiline mesylate by weight of the composition; 70% by weight of the composition of a mixture of a disintegrant, a flow agent and particles having a non-filamentous microstructure of at least two sugar alcohols; 21.6% xylitol by weight of the composition; 0.2% silicon dioxide by weight of the composition; 1.5% crosscarmelose sodium by weight of the composition; 2.8% starch by weight of the composition; 0.7% flavoring agent by weight of the composition; 0.3% sweetener by weight of the composition; and 2% sodium stearyl fumarate by weight of the composition.

This invention also provides a solid pharmaceutical composition comprising 2.1% rasagiline mesylate by weight of the composition; 63.3% by weight of the composition of a mixture of a disintegrant, a flow agent and particles having a non-filamentous microstructure of at least two sugar alcohols; 25.7% xylitol by weight of the composition; 0.3% silicon dioxide by weight of the composition; 1.7% crosscarmelose sodium by weight of the composition; 3.3% starch by weight of the composition; 1.1% flavoring agent by weight of the composition; 0.5% sweetener by weight of the composition; and 2% sodium stearyl fumarate by weight of the composition.

This invention also provides a solid pharmaceutical composition comprising 3.12 mg rasagiline mesylate; 245 mg of a mixture of a disintegrant, a flow agent and particles having a non-filamentous microstructure of at least two sugar alcohols; 77.276 mg of xylitol; 0.6 mg of silicon dioxide; 5.25 mg of crosscarmelose sodium; 10.0 mg of starch;
2.334 mg of a flavoring agent; 1.0 mg of a sweetener; and 6.8 mg of sodium stearyl fumarate.

This invention also provides a solid pharmaceutical composition comprising 3.12 mg rasagiline mesylate; 94.75 mg of a mixture of a disintegrant, a flow agent and particles having a non-filamentous microstructure of at least two sugar alcohols; 38.64 mg of xylitol; 0.45 mg of silicon dioxide; 2.265 mg of crosscarmelose sodium; 5.0 mg of starch; 1.665 mg of a flavoring agent; 0.75 mg of a sweetener; and 3.0 mg of sodium stearyl fumarate.

This invention also provides a solid pharmaceutical composition comprising rasagiline or a pharmaceutically acceptable salt of rasagiline and a sugar alcohol, which solid pharmaceutical composition disintegrates in the oral cavity of a human within 50 seconds.

This invention also provides a solid pharmaceutical composition comprising rasagiline or a pharmaceutically acceptable salt of rasagiline which is non-lyophilized, which solid pharmaceutical composition disintegrates in the oral cavity of a human within 50 seconds.

This invention also provides a solid pharmaceutical composition comprising rasagiline or a pharmaceutically acceptable salt of rasagiline which is free of lactose, which solid pharmaceutical composition disintegrates in the oral cavity of a human within 50 seconds.

This invention also provides a solid pharmaceutical composition comprising rasagiline or a pharmaceutically acceptable salt of rasagiline which is free of microcrystalline cellulose, which solid pharmaceutical composition disintegrates in the oral cavity of a human within 50 seconds.

This invention also provides a solid pharmaceutical composition comprising rasagiline or a pharmaceutically acceptable salt of rasagiline which is free of magnesium stearate, which solid pharmaceutical composition disintegrates in the oral cavity of a human within 50 seconds.

This invention further provides a method of treating a subject afflicted with Parkinson's disease comprising administering to the subject a therapeutically effective amount of the solid pharmaceutical composition, thereby treating the subject.

This invention provides a process of making a solid pharmaceutical composition comprising admixing rasagiline or a pharmaceutically acceptable salt of rasagiline, and a mixture of a disintegrant, a flow agent, and particles having a non-filamentous microstructure of at least two sugar alcohols.

This invention also provides process of making a solid pharmaceutical composition comprising admixing 3.12 mg rasagiline mesylate; 245 mg of a mixture of a disintegrant, a flow agent and particles having a non-filamentous microstructure of at least two sugar alcohols; 77.276 mg of xylitol; 0.6 mg of silicon dioxide; 5.25 mg of crosscarmelose sodium; 10.0 mg of starch; 2.334 mg of a flavoring agent; 1.0 mg of a sweetener; and 6.8 mg of sodium stearyl fumarate.

This invention further provides a process of making a solid pharmaceutical composition comprising admixing 3.12 mg rasagiline mesylate; 94.75 mg of a mixture of a disintegrant, a flow agent and particles having a non-filamentous microstructure of at least two sugar alcohols; 38.64 mg of xylitol; 0.45 mg of silicon dioxide; 2.265 mg of crosscarmelose sodium; 5.0 mg of starch; 1.665 mg of a flavoring agent; 0.75 mg of a sweetener; and 3.0 mg of sodium stearyl fumarate.

### DETAILED DESCRIPTION OF THE INVENTION

This invention provides a solid pharmaceutical composition comprising rasagiline or a pharmaceutically acceptable salt of rasagiline, and particles having a non-filamentous microstructure of at least two sugar alcohols.

In one embodiment, the at least two sugar alcohols are selected from a group consisting of mannitol, xylitol, sorbitol, maltitol and lactitol. In another embodiment, the at least two sugar alcohols are selected from a group consisting of mannitol, sorbitol, maltitol and xylitol. In yet another embodiment, the at least two sugar alcohols are mannitol and sorbitol.

In one embodiment, the amount of the particles having a non-filamentous microstructure is 50% to 75% by weight of the composition. In another embodiment, the amount of the particles having a non-filamentous microstructure is 50% to 70% by weight of the composition. In another embodiment, the amount of the particles having a non-filamentous microstructure is 50% to 65% by weight of the composition. In another embodiment, the amount of the particles having a non-filamentous microstructure is 50% to 60% by weight of the composition. In another embodiment, the amount of the particles having a non-filamentous microstructure is 55% to 75% by weight of the composition. In another embodiment, the amount of the particles having a non-filamentous microstructure is 55% to 70% by weight of the composition. In another embodiment, the amount of the particles having a non-filamentous microstructure is 55% to 60% by weight of the composition. In another embodiment, the amount of the particles having a non-filamentous microstructure is 55% to 65% by weight of the composition.

In one embodiment, the solid pharmaceutical composition further comprises a disintegrant. In one embodiment, the disintegrant is kaolin, powdered sugar, sodium starch glycolate, crosscarmelose sodium, carboxymethyl cellulose, microcrystalline cellulose, crosspovidone, sodium alginate, or a mixture of any of these. In another embodiment, the disintegrant is crosscarmelose sodium, crosspovidone, or a mixture of the two.

In one embodiment, the amount of disintegrant is from 5% to 15% by weight of the composition. In one embodiment, the amount of disintegrant is from 5% to 10% by weight of the composition. In one embodiment, the amount of disintegrant is from 10% to 15% by weight of the composition. In one embodiment, the amount of disintegrant is from 6% to 13% by weight of the composition. In one embodiment, the amount of disintegrant is from 7% to 10% by weight of the composition. In one embodiment, the amount of disintegrant is from 8% to 10% by weight of the composition. In one embodiment, the amount of disintegrant is from 7% to 9% by weight of the composition. In one embodiment, the amount of disintegrant is 8% by weight of the composition.

In one embodiment, the solid pharmaceutical composition further comprises a supplemental sugar alcohol. In one embodiment, the supplemental sugar alcohol is mannitol, xylitol, sorbitol, maltitol or lactitol. In another embodiment, the supplemental sugar alcohol is xylitol. In one embodiment, the amount of supplemental sugar alcohol is from 20% to 30% by weight of the composition.

In another embodiment, the solid pharmaceutical composition further comprises a lubricant. In one embodiment, the lubricant is sodium stearyl fumarate.

In one embodiment, the solid pharmaceutical composition is in the form of a tablet. In another embodiment, the solid pharmaceutical composition is in the form of a capsule, caplet, compressed pill, coated pill, dragee, sachet, hard gelatin capsule or dissolving strip.

In one embodiment, the solid pharmaceutical composition is characterized by a friability equal to or less than 1%. In one embodiment, the solid pharmaceutical composition is characterized by a friability equal to or less than 0.5%. In one embodiment, the solid pharmaceutical composition is characterized by a friability equal to or less than 0.2%.

In one embodiment, the solid pharmaceutical composition is in a non-lyophilized form.

In one embodiment, the solid pharmaceutical composition is free of lactose. In another embodiment, the solid pharmaceutical composition is free of microcrystalline cellulose. In yet another embodiment, the solid pharmaceutical composition is free of magnesium stearate.

In one embodiment, the solid pharmaceutical composition disintegrates in the oral cavity of a human within 50 seconds. In another embodiment, the solid pharmaceutical composition disintegrates in the oral cavity of a human within 45 seconds. In another embodiment, the solid pharmaceutical composition disintegrates in the oral cavity of a human within 40 seconds. In another embodiment, the solid pharmaceutical composition disintegrates in the oral cavity of a human within 35 seconds. In another embodiment, the solid pharmaceutical composition disintegrates in the oral cavity of a human within 30 seconds. In another embodiment, the solid pharmaceutical composition disintegrates in the oral cavity of a human within 25 seconds. In another embodiment, the solid pharmaceutical composition disintegrates in the oral cavity of a human within 20 seconds. In another embodiment, the solid pharmaceutical composition disintegrates in the oral cavity of a human within 15 seconds. In another embodiment, the solid pharmaceutical composition disintegrates in the oral cavity of a human within 10 seconds.

In one embodiment, the pharmaceutically acceptable salt of rasagiline is rasagiline mesylate.

This invention also provides a solid pharmaceutical composition comprising rasagiline or a pharmaceutically acceptable salt of rasagiline, a mixture of a disintegrant, a flow agent and particles having a non-filamentous microstructure of at least two sugar alcohols, a supplemental sugar alcohol, a supplemental flow agent, and a supplemental disintegrant.

In one embodiment, the at least two sugar alcohols of the particles having a non-filamentous microstructure are selected from a group consisting of mannitol, xylitol, sorbitol, maltitol and lactitol. In another embodiment, the at least two sugar alcohols of the particles having a non-filamentous microstructure are selected from a group consisting of mannitol, sorbitol, maltitol and xylitol. In yet another embodiment, the at least two sugar alcohols of the particles having a non-filamentous microstructure are mannitol and sorbitol.

In one embodiment, the amount of the particles having a non-filamentous microstructure is 50% to 75% by weight of the composition. In another embodiment, the amount of the particles having a non-filamentous microstructure is 55% to 65% by weight of the composition. In one embodiment, the supplemental disintegrant is kaolin, powdered sugar, sodium starch glycolate, crosscarmelose sodium, carboxymethyl cellulose, microcrystalline cellulose, crosspovidone, sodium alginate, or a mixture of any of these. In another embodiment, the disintegrant is crosspovidone and the supplemental disintegrant is crosscarmelose sodium. In one embodiment, the amount of supplemental disintegrant is from 0.5% to 5% by weight of the composition. In another embodiment, the amount of supplemental disintegrant is from 0.5% to 4.5% by weight of the composition. In another embodiment, the amount of supplemental disintegrant is from 0.5% to 4.0% by weight of the composition. In another embodiment, the amount of supplemental disintegrant is from 0.5% to 3.5% by weight of the composition. In another embodiment, the amount of supplemental disintegrant is from 0.5% to 3.0% by weight of the composition. In another embodiment, the amount of supplemental disintegrant is from 0.5% to 2.5% by weight of the composition. In another embodiment, the amount of supplemental disintegrant is from 0.5% to 2.0% by weight of the composition. In another embodiment, the amount of supplemental disintegrant is from 0.5% to 1.5% by weight of the composition. In another embodiment, the amount of supplemental disintegrant is from 1.0% to 4.5% by weight of the composition. In another embodiment, the amount of supplemental disintegrant is from 1.0% to 4.0% by weight of the composition. In another embodiment, the amount of supplemental disintegrant is from 1.0% to 3.5% by weight of the composition. In another embodiment, the amount of supplemental disintegrant is from 1.0% to 3.0% by weight of the composition. In another embodiment, the amount of supplemental disintegrant is from 1.0% to 2.5% by weight of the composition. In another embodiment, the amount of supplemental disintegrant is from 1.0% to 2.0% by weight of the composition. In another embodiment, the amount of supplemental disintegrant is from 1.0% to 1.5% by weight of the composition. In another embodiment, the amount of supplemental disintegrant is 1.5% by weight of the composition. In another embodiment, the amount of supplemental disintegrant is 1.7% by weight of the composition.

In one embodiment, the flow agent is silicon dioxide, and the supplemental flow agent is silicon dioxide. The flow agent may be colloidal silica, gel silica, precipitated silica or a combination thereof. In another embodiment, the amount of supplemental flow agent is from 0.1 to 1.0% by weight of the composition. In another embodiment, the amount of supplemental flow agent is from 0.1 to 0.9% by weight of the composition. In another embodiment, the amount of supplemental flow agent is from 0.1 to 0.8% by weight of the composition. In another embodiment, the amount of supplemental flow agent is from 0.1 to 0.7% by weight of the composition. In another embodiment, the amount of supplemental flow agent is from 0.1 to 0.6% by weight of the composition. In another embodiment, the amount of supplemental flow agent is from 0.1 to 0.5% by weight of the composition. In yet another embodiment, the amount of supplemental flow agent is 0.2% by weight of the composition. In yet another embodiment, the amount of supplemental flow agent is 0.3% by weight of the composition.

In one embodiment, the supplemental sugar alcohol is mannitol, xylitol, sorbitol, maltitol or lactitol. In yet another embodiment, the supplemental sugar alcohol is xylitol. In one embodiment, the amount of supplemental sugar alcohol is from 20% to 30% by weight of the composition. In yet another embodiment, the amount of supplemental sugar alcohol is 21.6% by weight of the composition. In yet another embodiment, the amount of supplemental sugar alcohol is 25.7% by weight of the composition.

In one embodiment, the solid pharmaceutical composition further comprises a lubricant. In one embodiment, the lubricant is sodium stearyl fumarate.

In one embodiment, the solid pharmaceutical composition is in the form of a tablet. In one embodiment, the solid pharmaceutical composition is in the form of a capsule, caplet, compressed pill, coated pill, dragee, sachet, hard gelatin capsule or dissolving strip.

In one embodiment, the solid pharmaceutical composition is characterized by a friability equal to or less than 1%. In one embodiment, the solid pharmaceutical composition is characterized by a friability equal to or less than 0.5%. In one embodiment, the solid pharmaceutical composition is characterized by a friability equal to or less than 0.2%.

In one embodiment, the solid pharmaceutical composition is in a non-lyophilized form. In another embodiment, the solid pharmaceutical composition is free of lactose. In another embodiment, the solid pharmaceutical composition is free of microcrystalline cellulose. In another embodiment, the solid pharmaceutical is free of magnesium stearate.

In one embodiment, the solid pharmaceutical composition disintegrates in the oral cavity of a human within 50 seconds. In another embodiment, the solid pharmaceutical composition disintegrates in the oral cavity of a human within 45 seconds. In another embodiment, the solid pharmaceutical composition disintegrates in the oral cavity of a human within 40 seconds. In another embodiment, the solid pharmaceutical composition disintegrates in the oral cavity of a human within 35 seconds. In another embodiment, the solid pharmaceutical composition disintegrates in the oral cavity of a human within 30 seconds. In another embodiment, the solid pharmaceutical composition disintegrates in the oral cavity of a human within 25 seconds. In another embodiment, the solid pharmaceutical composition disintegrates in the oral cavity of a human within 20 seconds. In another embodiment, the solid pharmaceutical composition disintegrates in the oral cavity of a human within 15 seconds. In another embodiment, the solid pharmaceutical composition disintegrates in the oral cavity of a human within 10 seconds.

In one embodiment, the pharmaceutically acceptable salt of rasagiline is rasagiline mesylate.

In one embodiment, the solid pharmaceutical composition is in unit dosage form comprising 1 mg of rasagiline. In one embodiment, the solid pharmaceutical composition is in unit dosage form comprising 2 mg of rasagiline. In one embodiment, the solid pharmaceutical composition is in unit dosage form comprising 1.56 mg of rasagiline mesylate. In one embodiment, the solid pharmaceutical composition is in unit dosage form comprising 3.12 mg of rasagiline mesylate.

The invention also provides a solid pharmaceutical composition comprising 0.9% rasagiline mesylate by weight of the composition; 70% by weight of the composition of a mixture of a disintegrant, a flow agent and particles having a non-filamentous microstructure of at least two sugar alcohols; 21.6% xylitol by weight of the composition; 0.2% silicon dioxide by weight of the composition; 1.5% crosscarmelose sodium by weight of the composition; 2.8% starch by weight of the composition; 0.7% flavoring agent by weight of the composition; 0.3% sweetener by weight of the composition; and 2% sodium stearyl fumarate by weight of the composition.

The invention also provides a solid pharmaceutical composition comprising 2.1% rasagiline mesylate by weight of the composition; 63.3% by weight of the composition of a mixture of a disintegrant, a flow agent and particles having a non-filamentous microstructure of at least two sugar alcohols; 25.7% xylitol by weight of the composition; 0.3% silicon dioxide by weight of the composition; 1.7% crosscarmelose sodium by weight of the composition; 3.3% starch by weight of the composition; 1.1% flavoring agent by weight of the composition; 0.5% sweetener by weight of the composition; and 2% sodium stearyl fumarate by weight of the composition.

This invention also provides a solid pharmaceutical composition comprising 3.12 mg rasagiline mesylate; 245 mg of a mixture of a disintegrant, a flow agent and particles having a non-filamentous microstructure of at least two sugar alcohols; 77.276 mg of xylitol; 0.6 mg of silicon dioxide; 5.25 mg of crosscarmelose sodium; 10.0 mg of starch; 2.334 mg of a flavoring agent; 1.0 mg of a sweetener; and 6.8 mg of sodium stearyl fumarate.

This invention also provides a solid pharmaceutical composition comprising 3.12 mg rasagiline mesylate; 94.75 mg of a mixture of a disintegrant, a flow agent and particles having a non-filamentous microstructure of at least two sugar alcohols; 38.64 mg of xylitol; 0.45 mg of silicon dioxide; 2.265 mg of crosscarmelose sodium; 5.0 mg of starch; 1.665 mg of a flavoring agent; 0.75 mg of a sweetener; and 3.0 mg of sodium stearyl fumarate.

This invention also provides a solid pharmaceutical composition comprising rasagiline or a pharmaceutically acceptable salt of rasagiline and a sugar alcohol, which solid pharmaceutical composition disintegrates in the oral cavity of a human within 50 seconds.

This invention also provides a solid pharmaceutical composition comprising rasagiline or a pharmaceutically acceptable salt of rasagiline which is non-lyophilized, which solid pharmaceutical composition disintegrates in the oral cavity of a human within 50 seconds.

This invention also provides a solid pharmaceutical composition comprising rasagiline or a pharmaceutically acceptable salt of rasagiline which is free of lactose, which solid pharmaceutical composition disintegrates in the oral cavity of a human within 50 seconds. In one embodiment, the solid pharmaceutical composition is in a non-lyophilized form.

This invention also provides a solid pharmaceutical composition comprising rasagiline or a pharmaceutically acceptable salt of rasagiline which is free of microcrystalline cellulose, which solid pharmaceutical composition disintegrates in the oral cavity of a human within 50 seconds. In one embodiment, the solid pharmaceutical composition is in a non-lyophilized form.

This invention also provides a solid pharmaceutical composition comprising rasagiline or a pharmaceutically acceptable salt of rasagiline which is free of magnesium stearate, which solid pharmaceutical composition disintegrates in the oral cavity of a human within 50 seconds. In one embodiment, the solid pharmaceutical composition is in a non-lyophilized form.

In one embodiment, the solid pharmaceutical composition has a hardness of 4-13 kPa.

In one embodiment, the particles of the solid pharmaceutical composition are co-processed particles of the at least two sugar alcohols. In another embodiment, the particles are co-spray dried particles of the at least two sugar alcohols.

This invention further provides a method of treating a subject afflicted with Parkinson's disease comprising administering to the subject a therapeutically effective amount of the solid pharmaceutical composition, thereby treating the subject.

This invention provides a process of making a solid pharmaceutical composition comprising admixing rasagiline or a pharmaceutically acceptable salt of rasagiline, and a mixture of a disintegrant, a flow agent, and particles having a non-filamentous microstructure of at least two sugar alcohols. In one embodiment, the process further comprises admixing a supplemental sugar alcohol, a supplemental flow agent and a supplemental disintegrant.

This invention also provides a process of making a solid pharmaceutical composition comprising admixing 3.12 mg rasagiline mesylate; 245 mg of a mixture of a disintegrant, a flow agent and particles having a non-filamentous microstructure of at least two sugar alcohols; 77.276 mg of xylitol; 0.6 mg of silicon dioxide; 5.25 mg of crosscarmelose sodium;10.0 mg of starch; 2.334 mg of a flavoring agent; 1.0 mg of a sweetener; and 6.8 mg of sodium stearyl fumarate.

This invention further provides a process of making a solid pharmaceutical composition comprising admixing 3.12 mg rasagiline mesylate; 94.75 mg of a mixture of a disintegrant, a flow agent and particles having a non-filamentous microstructure of at least two sugar alcohols; 38.64 mg of xylitol; 0.45 mg of silicon dioxide; 2.265 mg of crosscarmelose sodium; 5.0 mg of starch; 1.665 mg of a flavoring agent; 0.75 mg of a sweetener; and 3.0 mg of sodium stearyl fumarate.

All embodiments of the solid pharmaceutical composition described above may be embodiments of any solid pharmaceutical compositions of the present invention.

This invention provides a means to avoid the absorption of rasagiline in the stomach, and to eliminate the need for swallowing tablets, by absorption of rasagiline into the body before reaching the stomach. Such absorption of rasagiline can be accomplished by contact with the buccal, sublingual, pharyngeal and/or esophageal mucous membranes. To accomplish this, the invention discloses oral compositions designed to rapidly disperse within the mouth to allow maximum contact of rasagiline with the buccal, sublingual, pharyngeal and/or esophageal mucous membranes. Such compositions are not disclosed in the prior art formulations of rasagiline.

A pharmaceutically acceptable salt of rasagiline may be the mesylate, maleate, fumarate, tartrate, hydrobromide, esylate, p-toluenesulfonate, benzoate, acetate, phosphate or sulfate salt. In a preferred embodiment the salt is the mesylate, esylate or sulfate salt. In yet a more preferred embodiment, the salt is the mesylate salt.

Within the context of this application a "disintegrant" is an agent used in the pharmaceutical preparation of tablets, which causes them to disintegrate and release their medicinal substances on contact with moisture. Preferably, the tablets disintegrate rapidly in the mouth, within 50 seconds, preferably within 40 seconds, more preferably within 30 seconds, even more preferably within 20 seconds.

Within the context of this application, a "sugar alcohol" is defined as a polyhydric.alcohol having no more than one hydroxy group attached to each carbon atom, formed by the reduction of the carbonyl group of a sugar to a hydroxyl group. Examples of sugar alcohols include: mannitol, xylitol, sorbitol, maltitol and lactitol. Among other effects, sugar alcohols add to the pleasant taste of the compositions of the current invention, and allow for rapid disintegration in the mouth. Due to their endothermic dissolution properties, sugar alcohols also impart a cooling sensation in the mouth upon dissolution, and therefore aid in masking taste of bad tasting active ingredients and other excipients.

### Disintegration Enhancers

Excipients such as Pharmaburst^{™} C1 may be used to enhance disintegration rate. Pharmaburst^{™} is an easy-to-use quick dissolving delivery platform, which can be easily formulated with an active ingredient. Pharmaburst^{™} is a co-processed excipient system with specific excipients, which allows rapid disintegration and low adhesion to punch faces. The quantity of Pharmaburst^{™} required in a formulation will depend on the type of active ingredient and the desired quantity of the ingredient per tablet. Pharmaburst^{™} is smooth and creamy and helps to mask taste and grittiness of the active ingredients. Pharmaburst^{™} comprises Sugar Alcohols (like Mannitol, Maltitol, Sorbitol, Xylitol), Disintegrants (like Cross carmallose, crosspovidone) and Silicon dioxide.

Pharmaburst C1 is made using the following USP/EP excipients:

| Ingredients | Minimum | Maximum |
|---|---|---|
| Mannitol | 75% | 90% |
| Sorbitol | 6% | 20% |
| Crosspovidone (disintegrant) | 7% | 15% |
| Silicon dioxide (flow agent) | 0.1% | 1.5% |

Specific quick-dissolved excipients include co-spray-dried systems comprising sugar alcohols and disintegrants as disclosed in WO 03/051338. The following examples of quick-dissolving excipients systems for use in formulations for rapid dissolution are disclosed in International Application Publication WO 03/051338.

### Formulation Example No. 1:

A mixture of 547.48 grams of co-processed carbohydrate system consisting of mannitol and sorbitol in a 90:10 ratio (SPIPharma Inc. New Castle, DE), 61.00 grams of Polyplastadone-XL (ISP Technologies, Wayne, NJ) and 1.53 grams of Syloid® 244 FP (W.R. Grace & Co., Columbia MD) were blended in a Turbula Mixer for 10 minutes.

### Formulation Example No. 2:

A mixture of 547.48 grams of co-processed carbohydrate system consisting of mannitol and sorbitol in a 80:20 ratio (SPIPharma Inc. New Castle, DE), 61.00 grams of Polyplastadone-XL (ISP Technologies, Wayne, NJ) and 1.53 grams of Syloid® 244 FP (W.R. Grace & Co., Columbia MD) were blended in a Turbula Mixer for 10 minutes.

Within the context of this application, "co-processed" means the processing of at least two sugar alcohols together to make one product of particles having non-filamentous microstructures. A "co-processed carbohydrate" results from the processing of at least two polyols together to make a single product. A "co-processed carbohydrate system" is a co-processed carbohydrate and at least a disintegrant.

Polyplasdone XL-10 disintegrant is a synthetic, insoluble, but rapidly swellable, crosslinked, homopolymer of N-vinyl-2-pyrrolidone. It meets USP/NF, Ph Eur and JPE Pharmacopeial monographs for Crospovidone. Polyplasdone XL-10 disintegrant has a small particle size and narrow particle size distribution that impart a smooth mouth-feel to quick dissolve and chewable tablets. Large particles tend to result in a gritty mouth feel that many patients find objectionable. Therefore, smaller particles which are not felt in the mouth are preferred. When compared to other disintegrants, the average particle size of Polyplasdone XL-10 disintegrant is significantly lower. In addition, the narrow particle size distribution of Polyplasdone XL-10 disintegrant minimizes the presence of large particles that can cause a gritty mouth feel. These benefits are especially important in quick dissolve and chewable tablets that typically contain high levels of disintegrants. When introduced into water, Polyplasdone XL-10 disintegrant quickly wicks water into its capillaries and swells which results in rapid tablet disintegration.

Syloid® 244 FP silica is odorless, tasteless and meets the USP/NF and Food Chemical Codex (FCC) test requirements for Silicon Dioxide. Syloid® 244 FP silica is of the highest purity as it contains 99.6% SiO₂. Syloid® 244 FP has a high absorptive capacity, being able to absorb up to three times its weight in liquids. It is a micronized free flowing powder which is transparent and colorless in liquids. Syloid® 244 FP is insoluble except in HF and strong bases such as NaOH, and is completely inert.

Within the context of this application, "particles having non-filamentous microstructures" can be part of a compressed solid form, e.g. a tablet, wherein the particles having non-filamentous microstructures are agglomerated into such solid dosage forms by compression or compaction using standard tableting techniques. Agglomerated particles are thus referred to herein as "particles", which can closely clustered together in a compressed or compacted solid dosage form.

### Disintegration Test

The disintegration time in the mouth can be determined using the USP Disintegration Test for sublingual tablets disclosed on page 2302, section 701 of The United States Pharmacopeia. The National Formulary, Rockville MD., The United States Pharmacopeial Convention, Inc., 2004 Edition. This test is provided to determine compliance with the limits on disintegration stated in the individual monographs except where the label states that the tablets or capsules are intended for use as troches, or are to be chewed, or are designed as modified-release dosage forms (see The United States Pharmacopeia. The National Formulary, *Drug Release* <724>). For the purposes of this test, disintegration does not imply complete solution of the unit or even of its active constituent. Complete disintegration is defined as that state in which any residue of the unit, except fragments of insoluble coating or capsule shell, remaining on the screen of the test apparatus is a soft mass having no palpably firm core.

### Apparatus for USP Disintegration Test:

The apparatus consists of a basket-rack assembly, a 1000-mL, low-form beaker, 138 to 155 mm in height and having an inside diameter of 97 to 110 mm for the immersion fluid, a thermostatic arrangement for heating the fluid between 35° and 39°, and a device for raising and lowering the basket in the immersion fluid at a constant frequency rate between 29 and 32 cycles per minute through a distance of not less than 5.3 cm and not more than 5.7 cm. The volume of the fluid in the vessel is such that at that highest point of the upward stroke the wire mesh remains at least 2.5 cm below the surface of the fluid and descends to not less than 2.5 cm from the bottom of the vessel on the downward stroke. The time required for the upward stroke is equal to the time required for the downward stroke, and the change in stroke direction is a smooth transition, rather than an abrupt reversal of motion. The basket-rack assembly moves vertically along its axis. There is no appreciable horizontal motion or movement of the axis from the vertical.

**Basket-Rack Assembly**: The basket rack assembly consists of six open-ended transparent tubes, each 7.75±0.25 cm long and having an inside diameter of 20.7 to 23 mm and a wall 1.0 to 2.8 mm thick; the tubes are held in a vertical position by two plastic plates, each 8,8 to 9.2 cm in diameter and 5 to 7 mm in thickness, with six holes, each 22 to 26 mm in diameter, equidistant from the center of the plate and equally spaced from one another. Attached to the under surface of the lower plate is a woven stainless steel wire cloth, which has a plain square weave with 1.8- to 2.2-mm mesh apertures and with a wire diameter of 0.63±0.03 mm. The parts of the apparatus are assembled and rigidly held by means of three bolts passing through the two plastic plates. A suitable means is provided to suspend the basket-rack assembly from the raising and lowering device using a point on its axis. The design of the basket-rack assembly may be varied somewhat provided the specifications for the glass tubes and the screen mesh size are maintained.

**Disks:** The use of disks is permitted only where specified in the monograph. If specified in the individual monograph, each tube is provided with a cylindrical disk 9.5±0.15 mm thick and 20.7±0.15 mm in diameter. The disk is made of a suitable, transparent plastic material having a specific gravity of between 1.18 and 1.20. Five parallel 2mm holes extend between the ends of the cylinder. One of the holes is centered on the cylindrical axis. The other holes are centered 6mm from the axis on imaginary lines perpendicular to the axis and parallel to each other. Four identical trapezoidal-shaped planes are cut into the wall of the cylinder, nearly perpendicular to the ends of the cylinder. The trapezoidal shape is symmetrical; its parallel sides coincide with the ends of the cylinder and are parallel to an imaginary line connecting the centers of two adjacent holes 6 mm from the cylindrical axis. The parallel side of the trapezoid on the bottom of the cylinder has a length of 1.6mm, and its center lies at a depth of 1.8mm from the cylinder's circumference. The parallel side of the trapezoid on the top of the cylinder has a length of 9.4±0.2mm, and its center lies at a depth of 2.6±0.1 mm from the cylinder's circumference. All surfaces of the disk are smooth. If the use of disks is specified in the individual monograph, add a disk to each tube, and operate the apparatus as directed under the following procedure.

### Procedure for USP Disintegration Test:

**Uncoated Tablets-** Place 1 tablet in each of the six tubes of the basket and operate the apparatus, using water maintained at 37±2° as the immersion fluid unless otherwise specified in the individual monograph. At the end of the time limit specified in the monograph, lift the basket from the fluid, and observe the tables: all of the tablets have disintegrated completely. If 1 or 2 tablets fail to disintegrate completely, repeat the test on 12 additional tablets: not less than 16 of the total of 18 tablets tested disintegrate completely.

**Plain Coated Tablets**-Apply the test for *Uncoated Tablets,* operating the apparatus for the time specified in the individual monograph.

**Delayed-Release (Enteric Coated) Tablets**-Place 1 tablet in each of the six tubes of the basket and, if the tablet has a soluble external coating, immerse the basket in water at room temperature for 5 minutes. Operate the apparatus using simulate gastric fluid TS maintained at 37±2° as the immersion fluid. After 1 hour of operation in simulated gastric fluid TS, lift the basket from the fluid and observe the tablets: the tablets show no evidence of disintegration, cracking, or softening. Operate the apparatus, using simulated intestinal fluid TS maintained at 37±2° as the immersion fluid, for the time specified in the monograph. Lift the basket from the fluid, and observe the tablets: all of the tablets disintegrate completely. If 1 or 2 tablets fail to disintegrate completely, repeat the test on 12 additional tablets: not less than 16 of the total of 18 tablets tested disintegrate completely.

**Buccal Tablets**-Apply the test for *Uncoated Tablets.* After 4 hours, lift the basket from the fluid, and observe the tablets: all of the tablets have disintegrated. If 1 or 2 tablets fail to disintegrate completely, repeat the test on 12 additional tablets: not less than 16 of the total of 18 tablets tested disintegrate completely.

**Sublingual Tablets**-Apply the test for *Uncoated Tablets.* Observe the tablets within the time limit specified in the individual monograph: all of the tables have disintegrated. If 1 or 2 tablets fails to disintegrate completely, repeat the test on 12 additional tablets: not less than 16 of the total tablets tested disintegrate completely.

**Hard Gelatin Capsules**-Apply the test for *Uncoated Tablets.* Attach a removable wire cloth, which has a plain square weave with 1.8-2.2-mm mesh apertures and with a wire diameter of 0.60 to 0.655 mm, as described under *Basket-Rack Assembly,* to the surface of the upper plate of the basket-rack assembly. Observe the capsules within the time limit specified in the individual monograph: all of the capsules have disintegrated except for fragments from the capsule shell. If 1 or 2 capsules fail to disintegrate completely, repeat the test on 12 additional capsules: not less than 16 of the total of 18 capsules tested disintegrate completely.

### Friability

Within the context of this application, "friability" is defined as the tendency to crumble breaking into smaller particles. The friability is tested according to the USP Friability Test for tablets disclosed on pages 2621-2622, section 1216 of The United States Pharmacopeia. The National Formulary, Rockville MD., The United States Pharmacopeial Convention, Inc., 2004 Edition. This test provides guidelines for the friability determination of compressed, uncoated tablets. The test procedure presented in section 1216 is generally applicable to most compressed tablets.

The Friability Test method makes use of a drum, with an internal diameter between 283 and 291 mm and a depth between 36 and 40mm, of transparent synthetic polymer with polished internal surfaces, and not subject to static build-up. One side of the drum is removable. The tablets are tumbled at each turn of the drum by a curved projection with an inside radius between 75.5 and 85.5 mm that extends from the middle of the drum to outer wall. The drum is attached to the horizontal axis of a device that rotates at 25±1 rpm. Thus, at each turn the tablets roll or slide and fall onto the drum wall or onto each other. A drum with dual scooping supports for the running of two samples at one time may also be used.

For tablets with a unit mass equal to or less than 650 mg, a sample of whole tablets corresponding to 6.5 g is used. For tablets with a unit mass of more than 650mg, a sample of 10 whole tablets is used. The tablets are carefully de-dusted prior to testing. The tablet sample is accurately weighed, and placed in the drum. The drum is rotated 100 times, and the tablets are removed. The tablets are de-dusted as before, and accurately weighed.

Generally, the test is run once. If obviously cracked, cleaved, or broken tablets are present in the table sample after tumbling, the sample fails the test. If the results are doubtful or if the weight loss is greater than the targeted value, the test should be repeated twice and the mean of the three tests determined. A maximum weight loss of not more than 1% of the weight of the tablets being tested is considered acceptable for most products. In the case of new formulations, an initial weight loss of 0.8% would be permitted until sufficient packaging data are obtained to extend the limit to a targeted value of 1%.

If tablet size or shape causes irregular tumbling, adjust the drum base so that the bas forms an angle of about 10° with the bench top and the tablets no longer bind together when lying next to each other, which prevents them from falling freely.

Effervescent tablets and chewable tablets may have different specifications as far as friability is concerned, as these tablets normally require special packaging. In the case of hygroscopic tablets, a humidity-controlled environment (relative humidity less than 40%) is required for testing.

### Discussion

In order to ensure patient compliance, it is desirable to attain a pharmaceutical dosage form which has a pleasant taste, and disintegrates rapidly in the mouth, within e.g. 50 seconds. The disintegration time in the mouth can be determined using USP Disintegration Test for sublingual tablets disclosed on page 2302, section 701 of The United States Pharmacopeia. The National Formulary, Rockville MD., The United States Pharmacopeial Convention, Inc., 2004 Edition. If the pharmaceutical dosage form disintegrates in less than 50, 45, 40, 35, 30, 25, or 20 seconds using the USP Disintegration Test, it can be assumed that it will disintegrate in the oral cavity of a human in less than 50, 45, 40, 35, 30, 25, or 20 seconds, respectively.

An advantage of the tablets of this invention is that standard tableting procedures could be used in order to attain rasagiline orally dissolving tablets. There is no need for the timeconsuming, costly lyophilization process. In addition, the oral pharmaceutical compositions have a low friability (under 1%) and sufficient hardness and therefore can be packaged in standard containers, eliminating the need for special costly blister packages. Furthermore, the oral pharmaceutical compositions have a pleasant taste, and thereby patient compliance will be enhanced when these compositions are administered.

The cause of rasagiline instability in the formulations of the prior art has been attributed to the presence of at least one of microcrystalline cellulose, magnesium stearate, or lactose. The selection of the excipients used in the oral pharmaceutical compositions of the present invention accounts for this. Accordingly, a preferred embodiment of this invention uses quick-dissolving excipients such as Pharmaburst^{™}, which are free of any substantial amounts of any of microcrystalline cellulose, magnesium stearate or lactose.

### EXPERIMENTAL DETAILS

### Materials and Methods

Tablets A-E were prepared according to the following process. The excipients and active ingredients are listed in Table 1 below.

| Excipients | Function | mg/tablet | | | | |
|---|---|---|---|---|---|---|
| | | A | B | C | D | E |
| Rasagiline Mesylate | Active | 3.12 | 3.12 | 3.12 | 3.12 | 3.12 |
| Xylitol NF | Sugar alcohol | 77.276 | 77.276 | 227.276 | 77.276 | 38.64 |
| Aerosil 200 (Colloidal Silicon Dioxide NF/ EP) | Flow agent | 0.6 | 0.6 | 0.6 | 0.6 | 0.45 |
| Ac-Di-Sol (cross-carmelose Sodium NF) | Disintegrant | 5.25 | 5.25 | 5.25 | 5.25 | 2.625 |
| Starch NF/EP | Binder | 10.0 | 10.0 | 10.0 | 10.0 | 5.0 |
| Cherry Flavor #11929 SD | Flavoring Agent | 2.334 | 2.334 | 2.334 | 2.334 | 1.665 |
| Sodium Saccharin USP | Sweetener | 1.0 | 1.0 | 1.0 | 1.0 | 0.75 |
| Pharmaburst^{™} C1 | Co-spray dried Sugar Alcohol/ Disintegrant / flow agent | 245 | 245 | - | 245 | 94.75 |
| Sodium Bicarbonate | Disintegrant / Effervescent | - | - | 20 | - | - |
| Stearic Acid | Lubricant | 3.7 | 2.0 | 4.0 | - | - |
| Talc | Lubricant | 3.7 | 2.0 | 4.0 | - | - |
| Sodium Stearyl Fumarate | Lubricant | - | - | - | 6.8 | 3.0 |
| **Total tablet weight** | | **352** | **349** | **278** | **351** | **150** |

| | | | | | | |
|---|---|---|---|---|---|---|
| Note: 3.12 mg of Rasagiline Mesylate is equivalent to 2.0 mg of Rasagiline base. | | | | | | |

### Example 1

Formulation A was prepared using the excipients in Table 1 using the following steps:
1. Xylitol, 0.3 mg/tab aerosil, rasagiline mesylate, starch NF, Ac-Di-Sol, 1.34 mg/tab flavor, and 0.5 mg/tab sodium saccharin were mixed for 5 minutes.
2. Purified water USP was added to the mixture of step 1 and was mixed for 60 seconds.
3. The granulate was dried (outlet temp: 44°C).
4. The granulate was sieved through a 0.6 mesh screen.
5. The granulate was then mixed with 0.3 mg/tab aerosil, Pharmaburst^{™}, 0.5 mg/tab sodium saccharin, and 1 mg/tab cherry flavor for 15 minutes.
6. The mixture of step 5 was then mixed with stearic acid and talc for 5 minutes.
7. The tablets were pressed to a hardness of 5 kPa.

### Example 2

Formulation B was prepared using the excipients in Table 1 using the following steps:
1. Xylitol, 0.3 mg/tab aerosil, rasagiline mesylate, starch NF, Ac-Di-Sol, 1.34 mg/tab flavor, and 0.5 mg/tab sodium saccharin were mixed for 5 minutes.
2. Purified water USP was added to the mixture of step 1 and was mixed for 60 seconds.
3. The granulate was dried (outlet temp: 44°C).
4. The granulate was sieved through a 0.6 mesh screen.
5. The granulate was then mixed with 0.3 mg/tab aerosil, Pharmaburst^{™}, 0.5 mg/tab sodium saccharin, and 1 mg/tab cherry flavor for 15 minutes.
6. The mixture of step 5 was then mixed with stearic acid and talc for 5 minutes.
7. The tablets were pressed to a hardness of 6 kPa.

### Example 3

Formulation C was prepared using the excipients in Table 1 using the following steps:
1. 77.276 mg/tab xylitol, 0.3 mg/tab aerosil, rasagiline mesylate, starch NF, Ac-Di-Sol, 1.34 mg/tab flavor, and 0.5 mg/tab sodium saccharin were mixed for 5 minutes.
2. Purified water USP was added to the mixture of step 1 and was mixed for 60 seconds.
3. The granulate was dried (outlet temp: 44°C).
4. The granulate was sieved through a 0.6 mesh screen.
5. The granulate was then mixed with 0.3 mg/tab aerosil; sodium bicarbonate, 150 mg/tab xylitol, 0.5 mg/tab sodium saccharin, and 1 mg/tab cherry flavor for 15 minutes.
6. The mixture of step 5 was then mixed with stearic acid and talc for 5 minutes.
7. The tablets were pressed to a hardness of 4 kPa.

### Example 4

Formulation D was prepared using the excipients in Table 1 using the following steps:
1. Xylitol, 0.3 mg/tab aerosil, rasagiline mesylate, starch NF, Ac-Di-Sol, 1.34 mg/tab flavor, and 0.5 mg/tab sodium saccharin were mixed for 5 minutes.
2. Purified water USP was added to the mixture of step 1 and was mixed for 60 seconds.
3. The granulate was (outlet temp: 44°C).
4. The granulate was sieved through a 0.6 mesh screen.
5. The granulate was then mixed with 0.3 mg/tab aerosil, Pharmaburst^{™}, 0.5 mg/tab sodium saccharin, and 1 mg/tab cherry flavor for 15 minutes.
6. The mixture of step 5 was then mixed with sodium stearyl fumarate for 5 minutes.
7. The tablets were pressed to a hardness of 5 kPa.

### Example 5

Formulation E was prepared using the excipients in Table 1 using the following steps:
1. Xylitol, 0.15 mg/tab aerosil, rasagiline mesylate, starch NF, Ac-Di-Sol, 0.665 mg/tab flavor, and 0.25 mg/tab sodium saccharin were mixed for 5 minutes.
2. Purified water USP was added to the mixture of step 1 and was mixed for 50 seconds.
3. The granulate was dried (outlet temp: 44°C).
4. The granulate was sieved through a 0.6 mesh screen.
5. The granulate was then mixed with aerosil 0.3 mg/tab, Pharmaburst^{™}, 0.5 mg/tab sodium saccharin, and 1 mg/tab cherry flavor for 15 minutes.
6. The mixture of step 5 was then mixed with sodium stearyl fumarate for 5 minutes.
7. The tablets were pressed to a hardness of 5 kPa.

The taste of the tablets prepared according to formulation E was favorable.

### Example 6

Formulation F was prepared using the following excipients:

| Formulation F | Excipients |
|---|---|
| 0.78mg/tab | Rasagiline Mesylate |
| 79.62 mg/tab | Mannitol |
| 0.6 mg/tab | Aerosil 200 |
| 10.0 mg/tab | Starch 1500 |
| 10.0 mg/tab | Starch NF |
| 245 mg/tab | Pharmaburst C1 |
| 2.0 mg/tab | Stearic acid |
| 2.0 mg/tab | Talc USP |

| | |
|---|---|
| Note: 0.78 mg of Rasagiline Mesylate is equivalent to 0.5 mg of Rasagiline base. | |

1. Mannitol, 0.3mg/tab aerosil, rasagiline mesylate, starch NF, and starch 1500 were mixed for 5 minutes.
2. Purified water USP was poured onto the mixture of step 1 and mixed for 15 seconds.
3. The granulate was dried (outlet temp. 44°C).
4. The granulate was sieved through a 0.6 mesh screen.
5. The granulate was mixed with 0.3 mg/tab aerosil and Pharmaburst^{™} for 15 minutes.
6. The mixture of step 5 was then mixed with stearic acid and talc for 5 minutes.
7. The tablets were pressed to a hardness of 13 kPa.

The taste of the tablets prepared according to formulation F was not favorable.

### Example 7

### Disintegration Times and Friability: Table 2

The tablets were tested for disintegration time using USP Disintegration Test Method (section 701) as described above. The friability was tested according to USP Friability Test Method for tablets (section 1216) as described above.

**Table 2**

| Tablet Disintegration Times and Friability | | | | | | |
|---|---|---|---|---|---|---|
| Tablet | A | B | C | D | E | F |
| Disintegration Time (seconds) | 46 | 40 | 90 | 16 | 20 | 27 |
| Friability (percent) | 0.43 | 0.3 | No data | 0.37 | 0.1 | No Data |

## Claims

1. A solid pharmaceutical composition comprising rasagiline or a pharmaceutically acceptable salt of rasagiline, and particles having a non-filamentous microstructure of at least two sugar alcohols.

2. The solid pharmaceutical composition of claim 1 further comprising a disintegrant or supplemental disintegrant.

3. The solid pharmaceutical composition of any of claims 1 or 2 further comprising a supplemental sugar alcohol.

4. The solid pharmaceutical composition of claim 1 comprising
rasagiline or a pharmaceutically acceptable salt of rasagiline,
a mixture of a disintegrant, a flow agent and particles having a non-filamentous microstructure of at least two sugar alcohols,
a supplemental sugar alcohol,
a supplemental flow agent, and
a supplemental disintegrant.

5. The solid pharmaceutical composition of any one of claims 1-4, wherein the at least two sugar alcohols are selected from a group consisting of mannitol, xylitol, sorbitol, maltitol and lactitol.

6. The solid pharmaceutical composition of any one of claims 1-5, wherein the at least two sugar alcohols are mannitol and sorbitol.

7. The solid pharmaceutical composition of any one of claims 1-6, wherein the amount of the particles having a non-filamentous microstructure is 50% to 75% by weight of the composition.

8. The solid pharmaceutical composition of any one of claims 2-7, wherein the disintegrant or supplemental disintegrant is kaolin, powdered sugar, sodium starch glycolate, crosscarmelose sodium, carboxymethyl cellulose, microcrystalline cellulose, crosspovidone, sodium alginate, or a mixture of any of these.

9. The solid pharmaceutical composition of claim 8, wherein the disintegrant is crosspovidone, and the supplemental disintegrant is crosscarmelose sodium.

10. The solid pharmaceutical composition of any of claims 2-9, wherein the amount of supplemental disintegrant is from 0.5% to 5% by weight of the composition.

11. The solid pharmaceutical composition of claim 10, wherein the amount of supplemental disintegrant is 1.5% by weight of the composition; or is 1.7% by weight of the composition.

12. The solid pharmaceutical composition of any of claims 2-11, wherein the amount of disintegrant is from 5% to 15% by weight of the composition.

13. The solid pharmaceutical composition of claim 12, wherein the amount of disintegrant is 8% by weight of the composition.

14. The solid pharmaceutical composition of any one of claims 3-13, wherein the supplemental sugar alcohol is mannitol, xylitol, sorbitol, maltitol or lactitol.

15. The solid pharmaceutical composition of any one of claims 2-14, wherein the amount of supplemental sugar alcohol is from 20% to 30% by weight of the composition.

16. The solid pharmaceutical composition of any of claims 1-15 further comprising a lubricant.

17. The solid pharmaceutical composition of claim 16, wherein the lubricant is sodium stearyl fumarate.

18. The solid pharmaceutical composition of any one of claims 1-17 in the form of a tablet.

19. The solid pharmaceutical composition of any one of claims 1-17 in the form of a capsule, caplet, compressed pill, coated pill, dragee, sachet, hard gelatin capsule or dissolving strip.

20. The solid pharmaceutical composition of claim 18 with friability equal to or less than 1%.

21. The solid pharmaceutical composition of any of the claims 1-20 in a non-lyophilized form.

22. The solid pharmaceutical composition of any of the claims 1-21 which is free of lactose, free of microcrystalline cellulose, or free of magnesium stearate.

23. The solid pharmaceutical composition of any of claims 1-22, wherein the solid pharmaceutical composition disintegrates in the oral cavity of a human within 50 seconds.

24. The solid pharmaceutical composition of any of claims 1-23, comprising the pharmaceutically acceptable salt of rasagiline which is rasagiline mesylate.

25. The solid pharmaceutical composition of claim 4, wherein the flow agent is silicon dioxide, and the supplemental flow agent is silicon dioxide.

26. The solid pharmaceutical composition of any of claims 4, 9, 10 or 25, wherein the amount of supplemental flow agent is from 0.1 to 1.0% by weight of the composition.

27. The solid pharmaceutical composition of claim 26, wherein the amount of supplemental flow agent is 0.2% by weight of the composition, or is 0.3% by weight of the composition.

28. The solid pharmaceutical composition of claim 4, wherein the amount of supplemental sugar alcohol is 21.6% by weight of the composition, or is 25.7% by weight of the composition.

29. The solid pharmaceutical composition of any one of claims 1-28 in unit dosage form comprising 1 mg rasagiline, 2 mg rasagiline, 1.56 mg rasagiline mesylate or 3.12 mg of rasagiline mesylate.

30. The solid pharmaceutical composition of claim 4 comprising
0.9% rasagiline mesylate by weight of the composition;
70% by weight of the composition of a mixture of a disintegrant, a flow agent and particles having a non-filamentous microstructure of at least two sugar alcohols;
21.6% xylitol by weight of the composition;
0.2% silicon dioxide by weight of the composition;
1.5% crosscarmelose sodium by weight of the composition;
2.8% starch by weight of the composition;
0.7% flavoring agent by weight of the composition;
0.3% sweetener by weight of the composition; and
2% sodium stearyl fumarate by weight of the composition; or
comprising
2.1% rasagiline mesylate by weight of the composition;
63.3% by weight of the composition of a mixture of a disintegrant, a flow agent and particles having a non-filamentous microstructure of at least two sugar alcohols;
25.7% xylitol by weight of the composition;
0.3% silicon dioxide by weight of the composition;
1.7% crosscarmelose sodium by weight of the composition;
3.3% starch by weight of the composition;
1.1% flavoring agent by weight of the composition;
0.5% sweetener by weight of the composition; and
2% sodium stearyl fumarate by weight of the composition; or
comprising
3.12 mg rasagiline mesylate;
245 mg of a mixture of a disintegrant, a flow agent and particles having a non-filamentous microstructure of at least two sugar alcohols;
77.276 mg of xylitol;
0.6 mg of silicon dioxide;
5.25 mg of crosscarmelose sodium;
10.0 mg of starch;
2.334 mg of a flavoring agent;
1.0 mg of a sweetener; and
6.8 mg of sodium stearyl fumarate; or
comprising
3.12 mg rasagiline mesylate;
94.75 mg of a mixture of a disintegrant, a flow agent and particles having a non-filamentous microstructure of at least two sugar alcohols;
38.64 mg of xylitol;
0.45 mg of silicon dioxide;
2.265 mg of crosscarmelose sodium;
5.0 mg of starch;
1.665 mg of a flavoring agent;
0.75 mg of a sweetener; and
3.0 mg of sodium stearyl fumarate.

31. A solid pharmaceutical composition of any one of claims 18, 19, or 30 having a hardness of 4-13 kPa.

32. A solid pharmaceutical composition of any one of claims 1-30, wherein the particles are co-processed particles of the at least two sugar alcohols.

33. A solid pharmaceutical composition of claim 32, wherein the particles are co-spray dried particles of the at least two sugar alcohols.

34. Use of the solid pharmaceutical composition of any of the claims 1-33 for use in treating a subject afflicted with Parkinson's disease.

## Patentansprüche

1. Feste pharmazeutische Zusammensetzung umfassend Rasagilin oder ein pharmazeutisch verträgliches Salz von Rasagilin, und Partikel, die eine nicht-filamentöse Mikrostruktur von mindestens zwei Zuckeralkoholen aufweisen.

2. Feste pharmazeutische Zusammensetzung nach Anspruch 1 weiterhin umfassend ein Zerfallhilfsmittel oder ergänzendes Zerfallhilfsmittel.

3. Feste pharmazeutische Zusammensetzung nach einem der Ansprüche 1 oder 2 weiterhin umfassend einen ergänzenden Zuckeralkohol.

4. Feste pharmazeutische Zusammensetzung nach Anspruch 1 umfassend
Rasagilin oder ein pharmazeutisches verträgliches Salz von Rasagilin,
eine Mischung von einem Zerfallhilfsmittel, ein Fließmittel und Partikel, die eine nicht-filamentöse Mikrostruktur von mindestens zwei Zuckeralkoholen aufweisen,
einen ergänzenden Zuckeralkohol,
ein ergänzendes Fließmittel,
und ein ergänzendes Zerfallhilfsmittel.

5. Feste pharmazeutische Zusammensetzung nach einem der Ansprüche 1 - 4, wobei die mindestens zwei Zuckeralkohole ausgewählt sind aus einer Gruppe bestehend aus Mannit, Xylit, Sorbit, Malit und Lactit.

6. Feste pharmazeutische Zusammensetzung nach einem der Ansprüche 1-5, wobei die mindestens zwei Zuckeralkohole Mannit und Sorbit sind.

7. Feste pharmazeutische Zusammensetzung nach einem der Ansprüche 1-6, wobei die Menge der Partikel, die eine nicht-filamentöse Mikrostruktur aufweisen 50 Gew.-% bis 75 Gew.-% der Zusammensetzung ist.

8. Feste pharmazeutische Zusammensetzung nach einem der Ansprüche 2-7, wobei das Zerfallhilfsmittel oder ergänzende Zerfallhilfsmittel Kaolin, Puderzucker, Natriumstärkeglycolat, Natriumcrosscarmellose, Carboxymethylcellulose, mikrokristalline Cellulose, Crospovidon, Natriumalginat, oder eine beliebige Mischung aus diesen ist.

9. Feste pharmazeutische Zusammensetzung nach Anspruch 8, wobei das Zerfallhilfsmittel Crospovidon ist, und das ergänzende Zerfallhilfsmittel Natriumcrosscarmellose ist.

10. Feste pharmazeutische Zusammensetzung nach einem der Ansprüche 2-9, wobei die Menge an ergänzendem Zerfallhilfsmittel von 0.5 Gew.-% bis 5 Gew.-% der Zusammensetzung ist.

11. Feste pharmazeutische Zusammensetzung nach Anspruch 10, wobei die Menge an ergänzendem Zerfallhilfsmittel 1.5 Gew.-% der Zusammensetzung ist; oder 1.7 Gew.-% der Zusammensetzung ist.

12. Feste pharmazeutische Zusammensetzung nach einem der Ansprüche 2-11, wobei die Menge an Zerfallhilfsmittel von 5 Gew.-% bis 15 Gew.-% der Zusammensetzung ist.

13. Feste pharmazeutische Zusammensetzung nach Anspruch 12, wobei die Menge an Zerfallhilfsmittel 8 Gew.-% der Zusammensetzung ist.

14. Feste pharmazeutische Zusammensetzung nach einem der Ansprüche 3-13, wobei der ergänzende Zuckeralkohol Mannit, Xylit, Sorbit, Malit oder Lactit ist.

15. Feste pharmazeutische Zusammensetzung nach einem der Ansprüche 2-14, wobei die Menge an ergänzenden Zuckeralkohol von 20 Gew.-% bis 30 Gew.-% der Zusammensetzung ist.

16. Feste pharmazeutische Zusammensetzung nach einem der Ansprüche 1 bis 15 weiterhin umfassend ein Schmiermittel.

17. Feste pharmazeutische Zusammensetzung nach Anspruch 16, wobei das Schmiermittel Natriumstearylfumarat ist.

18. Feste pharmazeutische Zusammensetzung nach einem der Ansprüche 1 bis 17 in der Form einer Tablette.

19. Feste pharmazeutische Zusammensetzung nach einem der Ansprüche 1 bis 17 in der Form einer Kapsel, Filmtablette, verdichtete Pille, überzogene Pille, Dragee, Beutel, Hartgelatinkapsel oder sich auflösender Streifen.

20. Feste pharmazeutische Zusammensetzung nach Anspruch 18, mit einer Brüchigkeit gleich oder kleiner als 1 %.

21. Feste pharmazeutische Zusammensetzung nach einem der Ansprüche 1 bis 20 in einer nicht-lyophilisierten Form.

22. Feste pharmazeutische Zusammensetzung nach einem der Ansprüche 1 bis 21 welche frei von Laktose, frei von mikrokristalliner Cellulose, oder frei von Magnesiumstearat ist.

23. Feste pharmazeutische Zusammensetzung nach einem der Ansprüche 1 bis 22, wobei die feste pharmazeutische Zusammensetzung in der Mundhöhle eines Menschen innerhalb von 50 Sekunden zerfällt.

24. Feste pharmazeutische Zusammensetzung nach einem der Ansprüche 1 bis 23, umfassend das pharmazeutisch verträgliche Salz von Rasagilin, welches Rasagilinmesylat ist.

25. Feste pharmazeutische Zusammensetzung nach Anspruch 4, wobei das Fließmittel Silikondioxid, und das ergänzende Fließmittel Silikondioxid ist.

26. Feste pharmazeutische Zusammensetzung nach einem der Ansprüche 4, 9, 10 oder 25, wobei die Menge an ergänzendem Fließmittel von 0.1 bis 1.0 Gew.-% der Zusammensetzung ist.

27. Feste pharmazeutische Zusammensetzung nach Anspruch 26, wobei die Menge an ergänzendem Fließmittel 0.2 Gew.-% der Zusammensetzung ist, oder 0.3 Gew.-% der Zusammensetzung ist.

28. Feste pharmazeutische Zusammensetzung nach Anspruch 4, wobei die Menge an ergänzendem Zuckeralkohol 21.6 Gew.-% der Zusammensetzung ist, oder 25.7 Gew.-% der Zusammensetzung ist.

29. Feste pharmazeutische Zusammensetzung nach einem der Ansprüche 1 - 28 in Einheitsdosierungsform umfassend 1 mg Rasagilin, 2 mg Rasagilin, 1.56 mg Rasagilinmesylat oder 3.12 mg von Rasagilinmesylat.

30. Feste pharmazeutische Zusammensetzung nach Anspruch 4 umfassend
0.9 Gew.-% Rasagilinmesylat der Zusammensetzung;
70 Gew.-% der Zusammensetzung einer Mischung von einem Zerfallhilfsmittel, einem Fließmittel und Partikeln, die eine nicht-filamentöse Mikrostruktur von mindestens zwei Zuckeralkoholen aufweisen;
21.6 Gew.-% Xylit der Zusammensetzung;
0.2 Gew.-% Silikondioxid der Zusammensetzung;
1.5 Gew.-% Natriumcrosscarmellose der Zusammensetzung;
2.8 Gew.-% Stärke der Zusammensetzung;
0.7 Gew.-% Aromastoff der Zusammensetzung;
0.3 Gew.-% Süßstoff der Zusammensetzung; und
2 Gew.-% Natriumstearylfumarat der Zusammensetzung; oder
umfassend
2.1 Gew.-% Rasagilinmesylat der Zusammensetzung;
63.3 Gew.-% der Zusammensetzung einer Mischung von einem Zerfallhilfsmittel, einem Fließmittel und Partikeln die nicht-filamentöse Mikrostrukturen von mindestens zwei Zuckeralkoholen aufweisen;
25.7 Gew.-% Xylit der Zusammensetzung;
0.3 Gew.-% Silikondioxid der Zusammensetzung;
1.7 Gew.-% Natriumcrosscarmellose der Zusammensetzung;
3.3 Gew.-% Stärke der Zusammensetzung;
1.1 Gew.% Aromastoff der Zusammensetzung;
0.5 Gew.% Süßstoff der Zusammensetzung; und
2 Gew.-% Natriumstearylfumarat der Zusammensetzung; oder
umfassend
3.12 mg Rasagilinmesylat;
245 mg einer Mischung von einem Zerfallhilfsmittel, einem Fließmittel und Partikeln, die eine nicht-filamentöse Mikrostruktur von mindestens zwei Zuckeralkoholen aufweisen;
77.276 mg von Xylit;
0.6 mg von Silikondioxid;
5.25 mg von Natriumcrosscarmellose;
10.0 mg von Stärke;
2.334 mg von einem Aromastoff;
1.0 mg von einem Süßstoff; und
6.8 mg von Natriumstearylfumarat; oder
umfassend
3.12 mg Rasagilinmesylat;
94.75 mg einer Mischung von einem Zerfallhilfsmittel, einem Fließmittel und Partikeln, die eine nicht-filamentöse Mikrostruktur von mindestens zwei Zuckeralkoholen aufweisen;
38.64 mg von Xylit;
0.45 mg von Silikondioxid;
2.265 mg von Natriumcrosscarmellose;
5.0 mg von Stärke;
1.665 mg von einem Aromastoff;
0.75 mg von einem Süßstoff; und
3.0 mg von Natriumstearylfumarat.

31. Feste pharmazeutische Zusammensetzung nach einem der Ansprüche 18, 19, oder 30, die eine Härte von 4-13 kPa aufweist.

32. Feste pharmazeutische Zusammensetzung nach einem der Ansprüche 1 bis 30, wobei die Partikel gemeinsam verarbeitete (co-processed) Partikel der mindestens zwei Zuckeralkohole sind.

33. Feste pharmazeutische Zusammensetzung nach Anspruch 32, wobei die Partikel gemeinsam sprühgetrocknete (co-spray dried) Partikel der mindestens zwei Zuckeralkohole sind.

34. Verwendung der festen pharmazeutischen Zusammensetzung nach einem der Ansprüche 1-33 zur Verwendung in der Behandlung eines mit der Parkinson Krankheit belastetem Subjekt.

## Revendications

1. Composition pharmaceutique solide comprenant de la rasagiline ou un sel pharmaceutiquement acceptable de rasagiline, et des particules ayant une microstructure non filamenteuse d'au moins deux alcools de sucre.

2. Composition pharmaceutique solide selon la revendication 1, comprenant de plus un délitant ou un délitant supplémentaire.

3. Composition pharmaceutique solide selon l'une quelconque des revendications 1 ou 2, comprenant de plus un alcool de sucre supplémentaire.

4. Composition pharmaceutique solide selon la revendication 1, comprenant
de la rasagiline ou un sel pharmaceutiquement acceptable de rasagiline,
un mélange d'un délitant, d'un agent d'écoulement et de particules ayant une microstructure non filamenteuse d'au moins deux alcools de sucre,
un alcool de sucre supplémentaire,
un agent d'écoulement supplémentaire, et
un délitant supplémentaire.

5. Composition pharmaceutique solide selon l'une quelconque des revendications 1 à 4, dans laquelle les au moins deux alcools de sucre sont choisis dans un groupe constitué du mannitol, du xylitol, du sorbitol, du maltitol et du lactitol.

6. Composition pharmaceutique solide selon l'une quelconque des revendications 1 à 5, dans laquelle les au moins deux alcools de sucre sont du mannitol et du sorbitol.

7. Composition pharmaceutique solide selon l'une quelconque des revendications 1 à 6, dans laquelle la quantité des particules ayant une microstructure non filamenteuse va de 50 % à 75 % en poids de la composition.

8. Composition pharmaceutique solide selon l'une quelconque des revendications 2 à 7, dans laquelle le délitant ou le délitant supplémentaire est du kaolin, du sucre en poudre, du glycolate d'amidon sodique, de la croscarmellose sodique, de la carboxyméthylcellulose, de la cellulose microcristalline, de la crospovidone, de l'alginate de sodium, ou un mélange de n'importe lesquels d'entre eux.

9. Composition pharmaceutique solide selon la revendication 8, dans laquelle le délitant est de la crospovidone, et le délitant supplémentaire est de la croscarmellose sodique.

10. Composition pharmaceutique solide selon l'une quelconque des revendications 2 à 9, dans laquelle la quantité de délitant supplémentaire va de 0,5 % à 5 % en poids de la composition.

11. Composition pharmaceutique solide selon la revendication 10, dans laquelle la quantité de délitant supplémentaire est de 1,5 % en poids de la composition ; ou bien elle est de 1,7 % en poids de la composition.

12. Composition pharmaceutique solide selon l'une quelconque des revendications 2 à 11, dans laquelle la quantité de délitant va de 5 % à 15 % en poids de la composition.

13. Composition pharmaceutique solide selon la revendication 12, dans laquelle la quantité de délitant est de 8 % en poids de la composition.

14. Composition pharmaceutique solide selon l'une quelconque des revendications 3 à 13, dans laquelle l'alcool de sucre supplémentaire est du mannitol, du xylitol, du sorbitol, du maltitol ou du lactitol.

15. Composition pharmaceutique solide selon l'une quelconque des revendications 2 à 14, dans laquelle la quantité d'alcool de sucre supplémentaire va de 20 % à 30 % en poids de la composition.

16. Composition pharmaceutique solide selon l'une quelconque des revendications 1 à 15, comprenant de plus un lubrifiant.

17. Composition pharmaceutique solide selon la revendication 16, dans laquelle le lubrifiant est du stéarylfumarate de sodium.

18. Composition pharmaceutique solide selon l'une quelconque des revendications 1 à 17 sous la forme d'un comprimé.

19. Composition pharmaceutique solide selon l'une quelconque des revendications 1 à 17 sous la forme d'une capsule, d'une pastille, d'une pilule comprimée, d'une pilule enrobée, d'une dragée, d'un sachet, d'une capsule en gélatine dure (gélule) ou d'une bandelette à dissolution rapide.

20. Composition pharmaceutique solide selon la revendication 18 avec une friabilité égale ou inférieure à 1 %.

21. Composition pharmaceutique solide selon l'une quelconque des revendications 1 à 20 dans une forme non lyophilisée.

22. Composition pharmaceutique solide selon l'une quelconque des revendications 1 à 21, qui est exempte de lactose, exempte de cellulose microcristalline, ou exempte de stéarate de magnésium.

23. Composition pharmaceutique solide selon l'une quelconque des revendications 1 à 22, dans laquelle la composition pharmaceutique solide se délite en moins de 50 secondes dans la cavité orale d'un humain.

24. Composition pharmaceutique solide selon l'une quelconque des revendications 1 à 23, comprenant le sel pharmaceutiquement acceptable de rasagiline qui est du mésylate de rasagiline.

25. Composition pharmaceutique solide selon la revendication 4, dans laquelle l'agent d'écoulement est du dioxyde de silicium, et l'agent d'écoulement supplémentaire est du dioxyde de silicium.

26. Composition pharmaceutique solide selon l'une quelconque des revendications 4, 9, 10 ou 25, dans laquelle la quantité d'agent d'écoulement supplémentaire va de 0,1 à 1,0 % en poids de la composition.

27. Composition pharmaceutique solide selon la revendication 26, dans laquelle la quantité d'agent d'écoulement supplémentaire est de 0,2 % en poids de la composition, ou bien elle est de 0,3 % en poids de la composition.

28. Composition pharmaceutique solide selon la revendication 4, dans laquelle la quantité d'alcool de sucre supplémentaire est de 21,6 % en poids de la composition, ou bien elle est de 25,7 % en poids de la composition.

29. Composition pharmaceutique solide selon l'une quelconque des revendications 1 à 28 dans une forme de dosage unitaire comprenant 1 mg de rasagiline, 2 mg de rasagiline, 1,56 mg de mésylate de rasagiline ou 3,12 mg de mésylate de rasagiline.

30. Composition pharmaceutique solide selon la revendication 4, comprenant
du mésylate de rasagiline à 0,9 % en poids de la composition ;
70 % en poids de la composition d'un mélange d'un délitant, d'un agent d'écoulement et de particules ayant une microstructure non filamenteuse d'au moins deux alcools de sucre ;
du xylitol à 21,6 % en poids de la composition ;
du dioxyde de silicium à 0,2 % en poids de la composition ;
de la croscarmellose sodique à 1,5 % en poids de la composition ;
de l'amidon à 2,8 % en poids de la composition ;
un aromatisant à 0,7 % en poids de la composition ;
un édulcorant à 0,3 % en poids de la composition ; et
du stéarylfumarate de sodium à 2 % en poids de la composition ; ou
comprenant
du mésylate de rasagiline à 2,1 % en poids de la composition ;
63,3 % en poids de la composition d'un mélange d'un délitant, d'un agent d'écoulement et de particules ayant une microstructure non filamenteuse d'au moins deux alcools de sucre ;
du xylitol à 25,7 % en poids de la composition ;
du dioxyde de silicium à 0,3 % en poids de la composition ;
de la croscarmellose sodique à 1,7 % en poids de la composition ;
de l'amidon à 3,3 % en poids de la composition ;
un aromatisant à 1,1 % en poids de la composition ;
un édulcorant à 0,5 % en poids de la composition ; et du stéarylfumarate de sodium à 2 % en poids de la composition ; ou
comprenant
3,12 mg de mésylate de rasagiline ;
245 mg d'un mélange d'un délitant, d'un agent d'écoulement et de particules ayant une microstructure non filamenteuse d'au moins deux alcools de sucre ;
77,276 mg de xylitol ;
0,6 mg de dioxyde de silicium ;
5,25 mg de croscarmellose sodique ;
10,0 mg d'amidon ;
2,334 mg d'un aromatisant ;
1,0 mg d'un édulcorant ; et
6,8 mg de stéarylfumarate de sodium ; ou
comprenant
3,12 mg de mésylate de rasagiline ;
94,75 mg d'un mélange d'un délitant, d'un agent d'écoulement et de particules ayant une microstructure non filamenteuse d'au moins deux alcools de sucre ;
38,64 mg de xylitol ;
0,45 mg de dioxyde de silicium ;
2,265 mg de croscarmellose sodique ;
5,0 mg d'amidon ;
1,665 mg d'un aromatisant ;
0,75 mg d'un édulcorant ; et
3,0 mg de stéarylfumarate de sodium.

31. Composition pharmaceutique solide selon l'une quelconque des revendications 18, 19, ou 30 ayant une dureté de 4 à 13 kPa.

32. Composition pharmaceutique solide selon l'une quelconque des revendications 1 à 30, dans laquelle les particules sont des particules cotraitées des au moins deux alcools de sucre.

33. Composition pharmaceutique solide selon la revendication 32, dans laquelle les particules sont des particules coséchées par pulvérisation des au moins deux alcools de sucre.

34. Utilisation de la composition pharmaceutique solide selon l'une quelconque des revendications 1 à 33, pour une utilisation dans le traitement d'un sujet atteint de la maladie de Parkinson.
